# EUROPEAN PATENT APPLICATION

(11) **EP 4 759 917 A1**
(43) Date of publication of application: **17.06.2026**
(21) Application number: 25222342.5
(22) Date of filing: 10.12.2025
(51) Int. Cl.: C12M 1/00, C12M 1/26, C12N 5/0789, C12N 5/074

(54) **CELL CONCENTRATION APPARATUS AND METHOD FOR CONCENTRATING CELLS**

(30) Priority: 11.12.2024 JP 2024216137
(71) Applicant: Canon Kabushiki Kaisha, Tokyo, 146-8501 (JP); Canon Medical Systems Corporation, Tochigi 324-0036 (JP)
(72) Inventor: MIKI, Tsutomu, Tokyo 146-8501 (JP); HIGUCHI, Yuhi, Tokyo 146-8501 (JP)
(74) Representative: Marks & Clerk LLP

(57) **Abstract**

A cell concentration apparatus (1) according to an embodiment includes a first selection unit (51a,51b), a culture unit (48), and a second selection unit (52a,52b). The first selection unit (51a,51b) is configured to collect cells having a size equal to or larger than a first threshold from a plurality of cells contained in a liquid sample as a first cell group. The culture unit (48) configured to perform expansion culture of target cells included in the collected first cell group and serving as an object for concentration. The second selection unit (52a,52b) configured to select, from the expansion-cultured first cell group, cells having a size equal to or larger than a second threshold larger than the first threshold and collect the cells as a second cell group.

## Description

### FIELD

Embodiments described herein relate generally to a cell concentration apparatus and a method for concentrating cells.

### BACKGROUND

In recent years, induced pluripotent stem (iPS) cells have been attracting attention. The process of introducing initializing factors is necessary to establish the iPS cells. CD34-positive cells, which are hematopoietic progenitor cells, have been known to serve as the target cells for this factor introduction. Therefore, when the blood used as a material for the iPS cells contains a large number of the CD34-positive cells, introduction of the initializing factors can be efficiently performed.

### SUMMARY OF THE INVENTION

A cell concentration apparatus according to an embodiment includes: a first selection unit configured to collect cells having a size equal to or larger than a first threshold from a plurality of cells contained in a liquid sample as a first cell group; a culture unit configured to perform expansion culture of target cells included in the collected first cell group and serving as an object for concentration; and a second selection unit configured to select, from the expansion-cultured first cell group, cells having a size equal to or larger than a second threshold larger than the first threshold and collect the cells as a second cell group.

The liquid sample may be blood.

The target cells may be CD34-positive cells, the first selection unit may be configured to select a plurality of types of cells including the CD34-positive cells as the first cell group, the culture unit may be configured to perform expansion culture of the CD34-positive cells, and the second selection unit may be configured to select a plurality of cells including the expansion-cultured CD34-positive cells as the second cell group.

The culture unit may be configured to increase a size of the target cells and to proliferate the target cells by performing expansion culture in an environment corresponding to the target cells.

The first selection unit may be at least one of a filter passing particles having a particle size smaller than the first threshold and capturing particles having a particle size equal to or larger than the first threshold and a spiral flow path classifying particles based on a particle size, and the second selection unit may be at least one of a filter passing particles having a particle size smaller than the second threshold and capturing particles having a particle size equal to or larger than the second threshold and the spiral flow path.

The first selection unit may include the spiral flow path and is configured to select the first cell group by classifying particles having a particle size equal to or larger than the first threshold by fluid having a predetermined flow rate flowed into the spiral flow path, and the second selection unit may include the spiral flow path and is configured to select the second cell group by classifying particles having a particle size equal to or larger than the second threshold by fluid having a flow rate different from the predetermined flow rate using the spiral flow path.

A method for concentrating cells according to an embodiment includes: a first selection step of collecting cells having a size equal to or larger than a first threshold from a plurality of cells contained in a liquid sample as a first cell group; a culture step of performing expansion culture of target cells included in the collected first cell group and serving as an object for concentration, and a second selection step of selecting, from the expansion-cultured first cell group, cells having a size equal to or larger than a second threshold larger than the first threshold and collecting the cells as a second cell group.

The liquid sample may be blood.

The target cells may be CD34-positive cells; the first selection step may select a plurality of types of cells including the CD34-positive cells as the first cell group; the culture step may perform expansion culture of the CD34-positive cells, and the second selection step may select a plurality of cells including the expansion-cultured CD34-positive cells as the second cell group.

The culture step may increase a size of the target cells and proliferates the target cells by performing expansion culture in an environment corresponding to the target cells.

The first selection step may select the first cell group using at least one of a filter passing particles having a particle size smaller than the first threshold and capturing particles having a particle size equal to or larger than the first threshold and a spiral flow path classifying particles based on a particle size, and the second selection step may select the second cell group using at least one of a filter passing particles having a particle size smaller than the second threshold and capturing particles having a particle size equal to or larger than the second threshold and the spiral flow path.

The first selection step may select the first cell group by classifying particles having a particle size equal to or larger than the first threshold by controlling a flow rate of fluid flowed into the spiral flow path, and the second selection step may select the second cell group by classifying particles having a particle size equal to or larger than the second threshold by changing the flow rate from the flow rate of the first selection step using the spiral flow path used at the first selection step.

### BRIEF DESCRIPTION OF THE DRAWINGS

FIG. 1 a view illustrating an example of a schematic configuration of a cartridge used in a cell preparation apparatus according to an embodiment;
FIG. 2 is a view illustrating an example of a schematic configuration of the cell preparation apparatus according to the embodiment;
FIG. 3 is a view illustrating an example of an iPS cell preparation process (process) flow achieved by the cell preparation apparatus according to the embodiment;
FIG. 4 is a view illustrating an example of the flow of steps in a blood introduction process, a blood cell separation process, and an expansion culture process according to the embodiment;
FIG. 5 is a block diagram illustrating an example of a flow path configuration of the cartridge according to the embodiment;
FIG. 6 is a view illustrating an example of a liquid delivery route at a blood introduction step according to the embodiment;
FIG. 7 is a view illustrating an example of the liquid delivery route in a blood filtering step according to the embodiment;
FIG. 8 is a view illustrating an example of the liquid delivery route in a PBS rinse step according to the embodiment;
FIG. 9 is a view illustrating an example of the liquid delivery route in a cell collection step according to the embodiment;
FIG. 10 is a view illustrating an example of the liquid delivery route in a first classification step according to the embodiment;
FIG. 11 is a view illustrating an example of the liquid delivery route in a expansion culture step according to the embodiment;
FIG. 12 is a view illustrating an example of the liquid delivery route in a second classification step according to the embodiment; and
FIG. 13 is a view illustrating an example of the relationship between each step of the iPS cell preparation process according to the embodiment and the ratio of CD34-positive cells in a sample.

### DETAILED DESCRIPTION

A cell concentration apparatus according to an embodiment includes a first selection unit, a culture unit, and a second selection unit. The first selection unit is configured to collect cells having a size equal to or larger than a first threshold from a plurality of cells contained in a liquid sample as a first cell group. The culture unit configured to perform expansion culture of target cells included in the collected first cell group and serving as an object for concentration. The second selection unit configured to select, from the expansion-cultured first cell group, cells having a size equal to or larger than a second threshold larger than the first threshold and collect the cells as a second cell group.

Hereinafter, the embodiments of the cell concentration apparatus and method for concentrating cells will be described in detail with reference to the drawings.

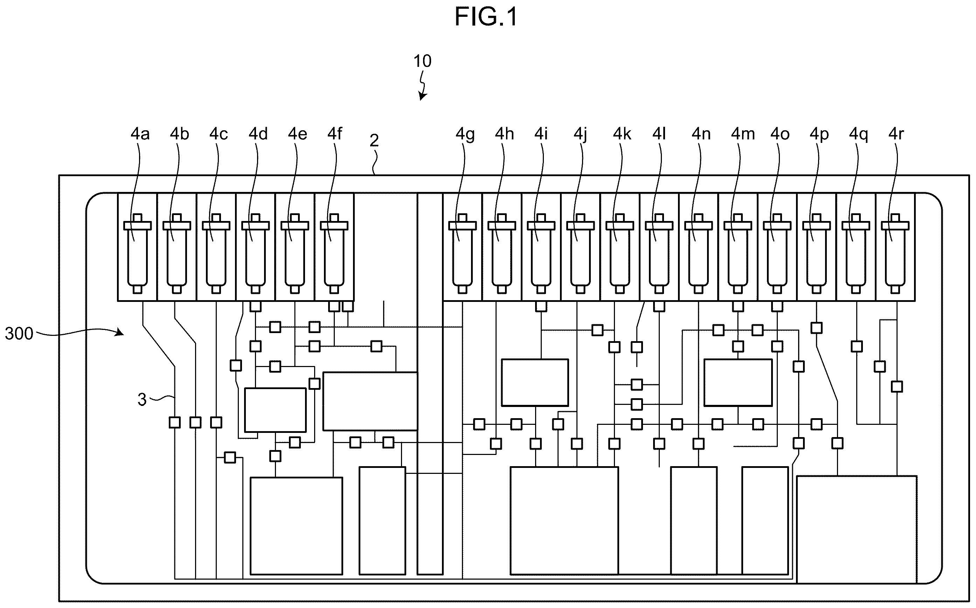

First, the configuration of the cell concentration apparatus according to the embodiment will be described. FIG. 1 is a view illustrating an example of a schematic configuration of a cartridge 10 used in the cell concentrating apparatus. The cartridge 10 consistently performs the entire processes of the cell preparation. In the present embodiment, the cartridge 10 is described, in particular, as preparing the induced pluripotent stem (iPS) cells.

As illustrated in FIG. 1, the cartridge 10 according to the present embodiment is a closed system apparatus including a cover 2, a pipe 3, and a plurality of containers 4a to 4r. The cartridge 10 according to the present embodiment is further includes a filter, a spiral flow path, and a drive reception unit, but these are omitted from the illustration in FIG. 1.

The containers 4a to 4r store various kinds of liquids used in the preparation of the iPS cells and liquids generated during the preparation of the iPS cell. The liquids used in the preparation of the iPS cells are samples and reagents. Specific examples include blood, cell suspension liquids, and saline solutions. For example, PBS (phosphate-buffered saline) may be used as the saline solution.

The sample used in the cartridge 10 is, for example, human blood. Blood is an example of the liquid sample. Of the containers 4a to 4r, those in which samples used for the iPS cell preparation are stored are called sample containers and those in which reagents are stored are called reagent containers. Specific examples of the sample containers include blood bags. Specific examples of the reagent containers include chemical solution bags.

The sample is not limited to blood, and may also be other cell mixture suspension liquids or the like. The liquid flowing through the flow path in the cartridge 10 according to the present embodiment is also called fluid.

Of the containers 4a to 4r, those other than the sample containers and the reagent containers such as blood bags and chemical solution bags are called liquid storage containers. Specific examples of the liquid storage containers include intermediate containers and collection containers. Hereafter, in the case where individual containers 4a to 4r are not specifically distinguished, these are simply called containers 4.

A plurality of pipes 3 are, for example, tubular components through which various liquids used in the preparation of the iPS cells flow. Silicone tubes, for example, are used for the pipes 3.

The pipes 3 and the containers 4 constitute a flow path 300 for delivering liquids used for the preparation of the iPS cells in the cartridge 10. In the cartridge 10 according to the present embodiment, the flow path 300 is a closed system flow path. Therefore, the flow path 300 of the cartridge 10 prevents leakage of liquids and substances outside of the flow path 300 and contamination of foreign substances from the outside.

The cover 2 covers the outside of the cartridge 10. Isolating the interior of the cartridge 10 from the external environment allows the cover 2 to prevent leakage outside the cartridge 10, even if the leakage of liquid or other substances should occur from the closed system flow paths in the cartridge 10. That is, the cartridge 10 has a double-closed structure of the cover 2 and the closed system flow path. The double-closed structure is also called a double-sealed structure.

The cartridge 10 is disposed after sterilization treatment after use, whereby the risk of infection caused by the blood or other substances used in the preparation of iPS cells is reduced. Of the components included in a cell preparation apparatus 1, the cartridge 10 is disposable. For example, the cartridge 10 is disposed after each single iPS cell preparation.

The other components (a liquid delivery apparatus 11, a valve opening/closing apparatus 12, a cooling apparatus 13, an incubator 14, and a transfer apparatus 15) can be used for a plurality of times of the iPS cell preparation. Usage in which the main unit of the cartridge 10 is used a plurality of times after replacing the pipes 3, the containers 4, the filters, and the like in the cartridge 10 may be applicable.

The number, the arrangement, and the shape of the containers 4a to 4r and pipes 3 illustrated in FIG. 1 are examples and are not limited thereto.

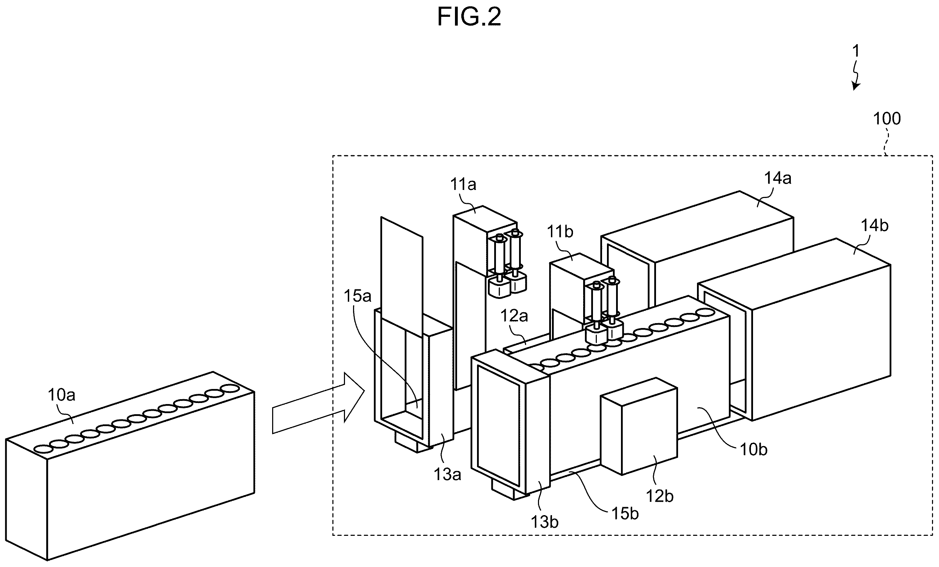

FIG. 2 is a view illustrating an example of the schematic configuration of the cell preparation apparatus 1. The cell preparation apparatus 1 is an example of a cell concentration apparatus. As illustrated in FIG. 2, the cell preparation apparatus 1 includes cartridges 10a and 10b, liquid delivery apparatuses 11a and 11b, valve opening/closing apparatuses 12a and 12b, cooling apparatuses 13a and 13b, incubators 14a and 14b, and transfer apparatuses 15a and 15b. The cell preparation apparatus 1 also includes a processing circuitry that controls the entire cell preparation apparatus 1.

The liquid delivery apparatuses 11a and 11b drive the liquid delivery in the cartridges 10a and 10b by applying pressurization or depressurization of gas pressure to the liquid storage containers of the cartridges 10a and 10b.

For example, the liquid delivery apparatuses 11a and 11b include regulators controlling the strength of the gas pressure to be pressurized or depressurized, pressure pads transmitting pressure to the cartridges 10a and 10b, and transfer mechanisms transferring the pressure pads to the liquid storage containers that are objects to be pressurized or depressurized. The liquid delivery apparatuses 11a and 11b may further include computers or other apparatuses for liquid delivery control.

The valve opening/closing apparatuses 12a and 12b open and close valves equipped with the pipes of the cartridges 10a and 10b, whereby the liquid delivery routes in the cartridges 10a and 10b are changed.

The cooling apparatuses 13a and 13b cool the samples and the reagents previously filled in the cartridges 10a and 10b to suitable temperatures. The cooling apparatuses 13a and 13b can be opened and closed, and the cartridges 10a and 10b are inserted by the operator through the openings in the cooling apparatuses 13a and 13b into a main unit 100 of the cell preparation apparatus 1. The cartridge 10 is previously filled with the samples and the reagents by the operator prior to the insertion into the cell preparation apparatus 1.

The incubators 14a and 14b maintain a suitable environment for cell culture by keeping, for example, the temperature, humidity, and carbon dioxide concentration in the cartridges 10a and 10b constant. During the cell culture, the cartridges 10a and 10b are inserted into the incubators 14a and 14b by the transfer apparatuses 15a and 15b.

The transfer apparatuses 15a and 15b transfer the position of the cartridges 10a and 10b in the main unit 100 of the cell preparation apparatus 1.

In the example illustrated in FIG. 2, one cell preparation apparatus 1 includes two cartridges 10a and 10b, two liquid delivery apparatuses 11a and 11b, two valve opening/closing apparatuses 12a and 12b, two cooling apparatuses 13a and 13b, two incubators 14a and 14b, and two transfer apparatuses 15a and 15b. This configuration, however, is only one example and is not limited thereto.

For example, the cell preparation apparatus 1 may include one of each apparatus, or may include three or more apparatuses. Hereinafter, in the case where the two cartridges 10a and 10b, the two liquid delivery apparatuses 11a and 11b, the two valve opening/closing apparatuses 12a and 12b, the two cooling apparatuses 13a and 13b, the two incubators 14a and 14b, and the two transfer apparatuses 15a and 15b are not particularly distinguished, these will be simply called the cartridge 10, the liquid delivery apparatus 11, the valve opening/closing apparatus 12, the cooling apparatus 13, the incubator 14, and the transfer apparatus 15.

The cell preparation apparatus 1 may be further include control apparatuses and other apparatuses other than the apparatuses illustrated in FIG. 2. The cell preparation apparatus 1 according to the present embodiment shall include at least the cartridge 10 and the liquid delivery apparatus 11.

Subsequently, the iPS cell preparation process performed by the cell preparation apparatus 1 will be described. The iPS cells are prepared by introducing specific genes into somatic cells and thereafter culturing, and have the ability to differentiate into cells of various tissues and organs. In the present embodiment, nucleated cells among the somatic cells, in particular, leukocytes shall be used as an iPS cell material.

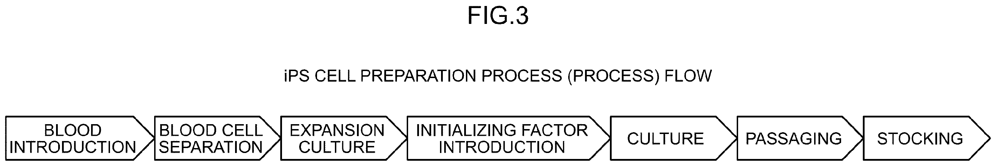

FIG. 3 is a view illustrating an example of the iPS cell preparation process (process) flow achieved by the cell preparation apparatus 1 according to the present embodiment. As illustrated in FIG. 3, the iPS cell preparation includes a blood introduction process, a blood cell separation process, an expansion culture process, an initializing factor introduction process, a culture process, a passaging process, and a stocking process.

The blood introduction process is a process of introducing blood containing leukocytes, which is the material for the iPS cells, into the cell preparation apparatus 1.

The blood cell separation process is a process to select leukocytes in the blood introduced into the cell preparation apparatus 1 and furthermore, to extract only specific cells (hereinafter may also called "specific cells") from the selected leukocytes. For example, the specific cells are the leukocytes of which nucleus inside are not broken among leukocytes. In the present embodiment, the specific cells include cluster of differentiation 34 (CD34)-positive cells that are targets for the initiating factor introduction.

The expansion culture process is a process to increase in size of the specific cells and proliferate the specific cells in the isolated leukocytes.

The initializing factor introduction process is a process to introduce the initializing factors into the expansion-cultured specific cells. In the present embodiment, the CD34-positive cells are introduced with the initializing factors at this process.

The culture process is a process to proliferate the cells (iPS cells) to which the initializing factors are introduced in a culture container.

The passaging process is a process to transfer the iPS cells proliferated at the culture process to a new culture container and to continuously proliferate the iPS cells.

The stocking process is a process to cryopreserve the iPS cells subcultured at the passaging process.

In the following description, the individual process related to the cell preparation illustrated in FIG. 3 is also called "process". In addition, each process includes one or more single operations to flow a liquid such as blood or a saline solution. In the following description, individual liquid delivery operation included in the process is also called "step" or "liquid delivery step".

Although all of the processes illustrated in FIG. 3 are performed by the cell preparation apparatus 1, the blood introduction process, the blood cell separation process, and the expansion culture process are particularly illustrated as examples in the present embodiment.

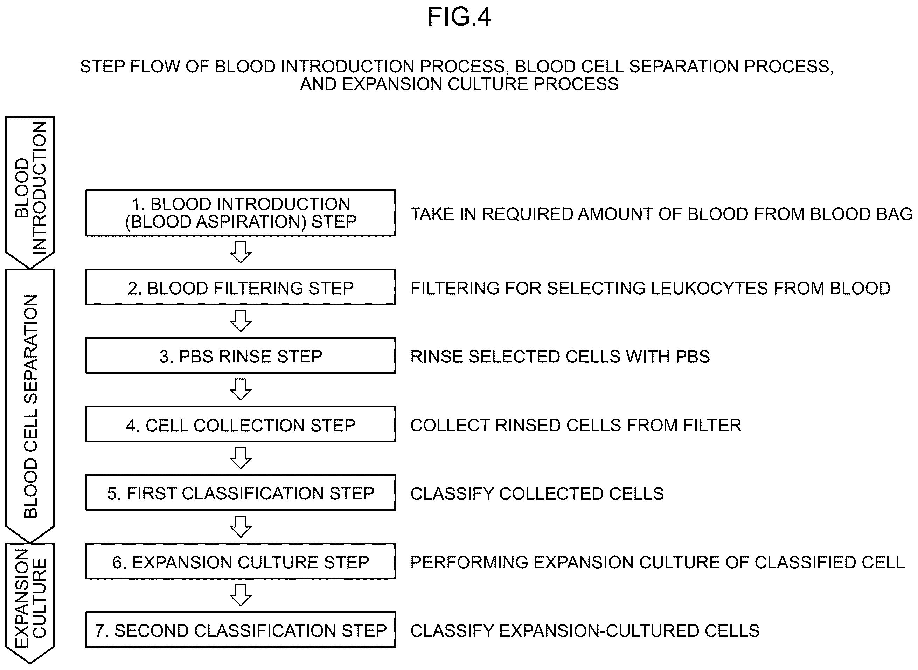

FIG. 4 is a view illustrating an example of the flow of the steps in the blood introduction process, blood cell separation process, and expansion culture process according to the embodiment.

As illustrated in FIG. 4, the blood introduction process includes a blood introduction (blood aspiration) step. As an example, the blood cell separation process also includes four steps: a blood filtering step, a PBS rinse step, a cell collection step, and a first classification step. As an example, the expansion culture process includes two steps: an expansion culture step and a second classification step.

In FIG. 4, the seven steps included in the blood introduction process, the blood cell separation process, and the expansion culture process are described as a series of steps.

The first blood introduction step is a liquid delivery operation introducing the required amount of blood into the pipe 3 of the flow path 300. In other words, the blood introduction step is also called a blood aspiration step because the step is a liquid delivery operation in which the required amount of blood is taken in from the blood bag into the pipe 3 of the flow path 300.

The second blood filtering step is a liquid delivery operation in which blood is passed through a filter to select leukocytes from the blood. Since the particle size of selection in the blood filtering step is rougher than that of the later first classification step, the results of the selection in this stage may include not only the specific cells to serve as an object for the selection but also other blood cells.

The third PBS rinse step is a liquid delivery operation in which cells selected by the filter are rinsed with PBS.

The fourth cell collection step is a liquid delivery operation that collects the rinsed cells from the filter.

The fifth first classification step is a liquid delivery operation that the cells collected from the filter are classified. These steps allow the cells other than the specific cells that cannot be fully removed at the blood filtering step to be removed and the specific cells to be extracted.

The sixth expansion culture step is a process of expansion-culturing the extracted specific cells. In the present embodiment, this allows the CD34-positive cells, which are the target of the initializing factor introduction at the subsequent initializing factor introduction process, to increase in size and to proliferate.

The seventh second classification step is a liquid delivery operation that classifies the expansion-cultured specific cells. This step is different from the fifth first classification step in terms of the flow rate of the liquid delivery. This allows a cell group containing a large number of CD34-positive cells to be extracted.

Here, the flow path 300 that achieves each of the steps illustrated in FIG. 4 will be described.

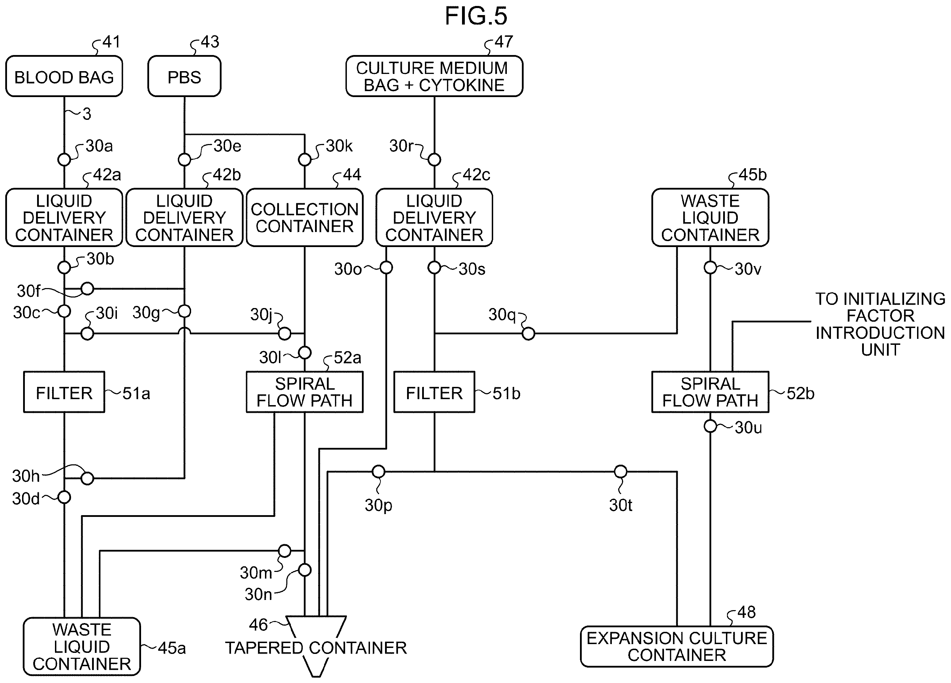

FIG. 5 is a view illustrating an example of a flow path configuration included in the cartridge 10 according to the embodiment that achieves the blood introduction process, the blood cell separation process, and the expansion culture process. The cartridge 10 is an example of a cell concentration apparatus.

As illustrated in FIG. 5, a flow path 300a includes a plurality of pipes 3, valves 30a to 30v, filters 51a and 51b, spiral flow paths 52a and 52b, a blood bag 41, liquid delivery containers 42 (42a to 42c), a chemical solution bag 43, a collection container 44, waste liquid containers 45 (45a and 45b), a tapered container 46, a culture medium bag 47, and an expansion culture container 48. The filter 51a and the spiral flow path 52a are examples of the first selection unit and the filter 51b and the spiral flow path 52b are examples of the second selection unit.

The blood bag 41, the liquid delivery containers 42a and 42b, the chemical solution bag 43, the collection container 44, and the culture medium bag 47 illustrated in FIG. 5 are examples of the containers 4 included in the cartridge 10.

In the present embodiment, the route through which the liquid flows in the flow path 300a by the step-by-step liquid delivery operation is called the liquid delivery route. The liquid delivery route varies from step to step.

The blood bag 41 contains blood that is used as a material for the iPS cells. The blood bag 41 is attached to the cartridge 10 by an operator in a state where blood is contained.

The liquid delivery containers 42a and 42b temporarily store blood or the chemical solutions such as PBS in the middle of the flow path 300a.

The chemical solution bag 43 is attached to the cartridge 10 by an operator in a state where a chemical solution such as a saline solution used in the preparation of the iPS cells is contained. In the present embodiment, the chemical solution bag 43 contains PBS.

The collection container 44 stores the cells collected from the filter 51a.

The waste liquid container 45a stores blood cells other than the specific cells classified from the cells collected from the filter 51a.

The tapered container 46 stores the classified specific cells at the end of the blood cell separation process. The cell suspension liquid containing cells stored in the tapered container 46 is delivered to the expansion culture container 48.

The culture medium bag 47 contains a chemical solution such as a culture medium used for the expansion culture.

This chemical solution is used to create an environment suitable for culturing the target cells serving as the object for concentration. In the present embodiment, the target cells are the CD34-positive cells. The culture medium bag 47 is attached to the cartridge 10 by the operator in a state where the chemical solution such as the culture medium is contained. In the present embodiment, the culture medium bag 47 contains a liquid culture medium and cytokines suitable for culturing the CD34-positive cells.

The expansion culture container 48 is a culture container for performing the expansion culture of the cells contained in the cell suspension liquid delivered from the tapered container 46.

The pipe 3 is a tubular component having holes for liquid flow. The pipe 3 connects between the filters 51a and 51b, the spiral flow paths 52a and 52b, the blood bag 41, the liquid delivery containers 42a and 42b, the chemical solution bag 43, the collection container 44, the waste liquid containers 45a and 45b, the tapered container 46, the culture medium bag 47, and the expansion culture container 48.

As the pipe 3, for example, a silicon tube can be applied as described above. In the present embodiment, the size of the pipe 3 is about 3 mm in an outer diameter and about 1 mm in an inner diameter as an example. The connecting portion of the pipe 3 is connected by, for example, a T-joint.

The valves 30a to 30v are provided in the pipe 3 and open and close the flow paths under the control of the valve opening/closing apparatus 12 to change the liquid delivery route in the flow path 300a.

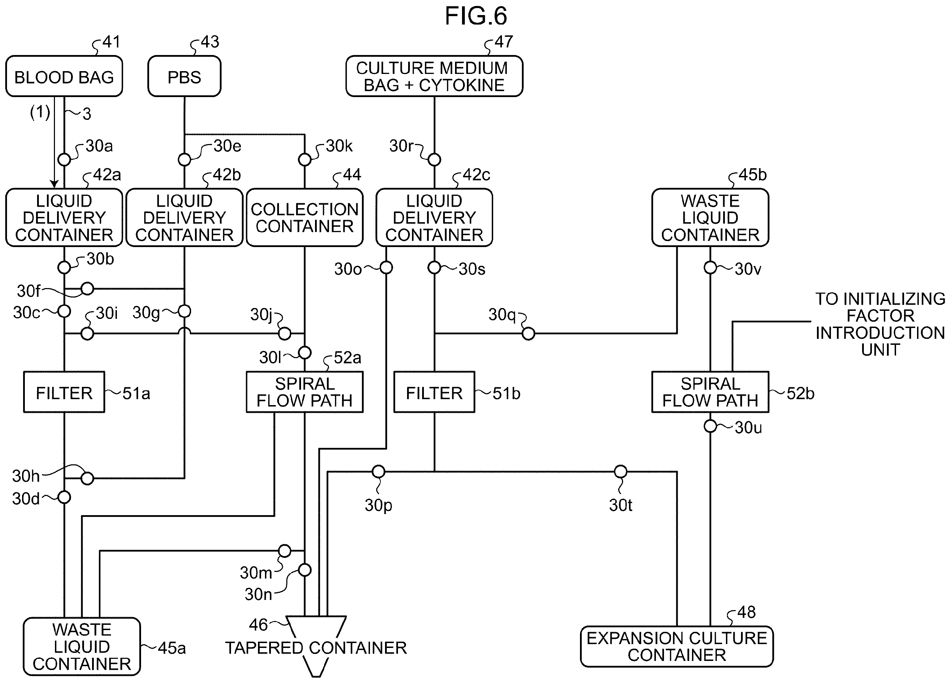

Hereinafter, each of the steps illustrated in FIG. 4, which is achieved by the flow path 300 configured as described above, will be described . First, the blood introduction step will be described. As a premise, all valves 30a to 30u are set in a closed state before the start of the blood introduction step. FIG. 6 is a view illustrating an example of a liquid delivery route in the blood introduction step. In FIG. 6, the liquid delivery route in the blood introduction step is represented by (1) and an arrow.

In the blood introduction step, the valve opening/closing apparatus 12 first sets the valve 30a in an open state. Thereafter, the liquid delivery apparatus 11 depressurizes the liquid delivery container 42a. This allows blood to be delivered from the blood bag 41 to the liquid delivery container 42a. As described above, the liquid delivery apparatus 11 depressurizes the inflow side of the liquid delivery container 42a, whereby liquid delivery control can be performed to the blood stored in the blood bag 41.

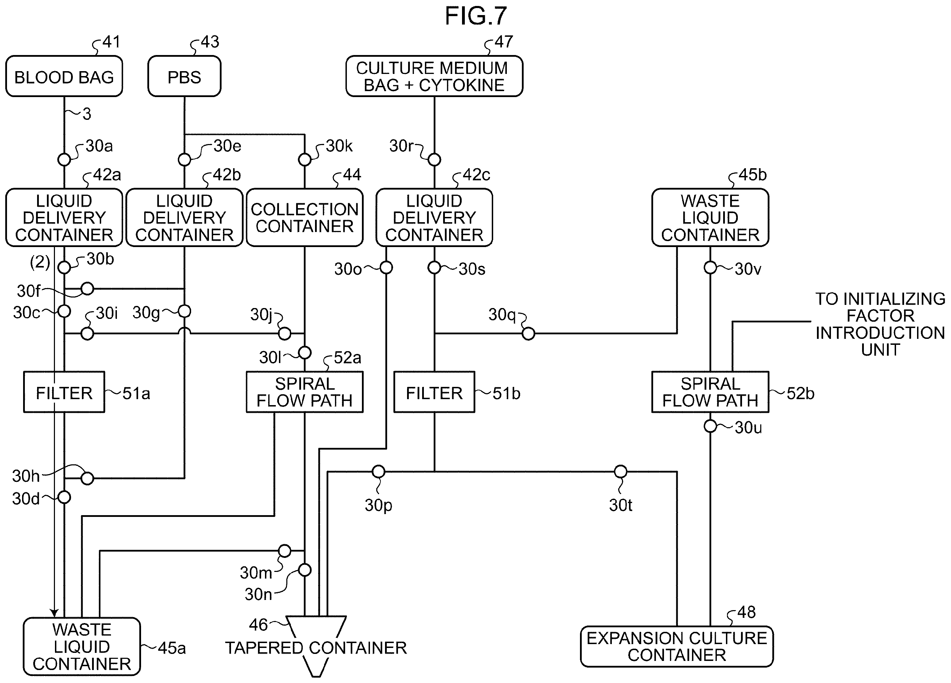

Subsequently, the blood filtering step will be described. FIG. 7 is a view illustrating an example of a liquid delivery route in the blood filtering step. In FIG. 7, the liquid delivery route in the blood filtering step is represented by (2) and an arrow.

At the blood filtering step, the valve opening/closing apparatus 12 sets the valve 30a in the closed state and the valves 30b, 30c, and 30d in the open state. Thereafter, the liquid delivery apparatus 11 pressurizes the liquid delivery container 42a. This allows blood to be delivered from the liquid delivery container 42a through the filter 51a to the waste liquid container 45a.

The filter 51a selects objects from the liquid flowing in the flow path 300a. The filter 51a is configured so as to pass particles having a size smaller than a separation threshold A and to capture particles having a size equal to or larger the separation threshold A. The separation threshold A is an example of the first threshold. In the case where the iPS cells are prepared from blood, the separation threshold A is preferably set to about 6 µm in order to properly remove red blood cells and platelets in blood.

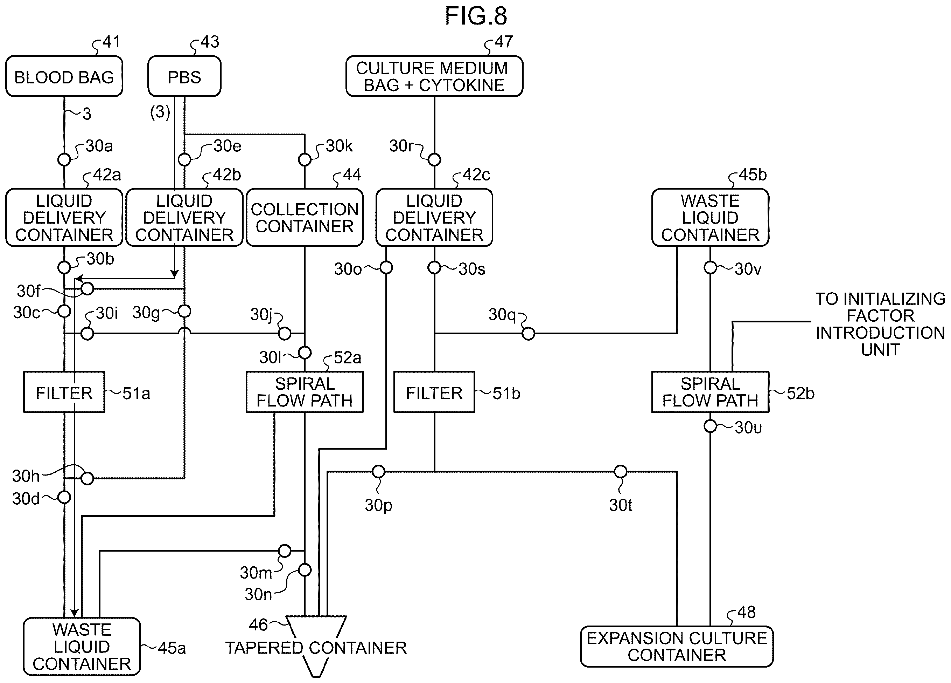

Subsequently, the PBS rinse step will be described. FIG. 8 is a view illustrating an example of a liquid delivery route in the PBS rinse step. In FIG. 8, the liquid delivery route in the PBS rinse step is represented by (3) and arrows.

At the PBS rinse step, the valve opening/closing apparatus 12 first sets the valve 30e in the open state. Thereafter, the liquid delivery apparatus 11 depressurizes the liquid delivery container 42b. This allows PBS to be delivered from the chemical solution bag 43 to the liquid delivery container 42b. As described above, the liquid delivery apparatus 11 depressurizes the liquid delivery container 42b on the inflow side, whereby the liquid delivery control for the PBS stored in the chemical solution bag 43 can be performed.

After the PBS is delivered into the liquid delivery container 42b, the valve opening/closing apparatus 12 sets the valve 30f in the open state. Thereafter, the liquid delivery apparatus 11 pressurizes the liquid delivery container 42b. This allows the PBS to be delivered from the liquid delivery container 42b through the filter 51a to the waste liquid container 45a.

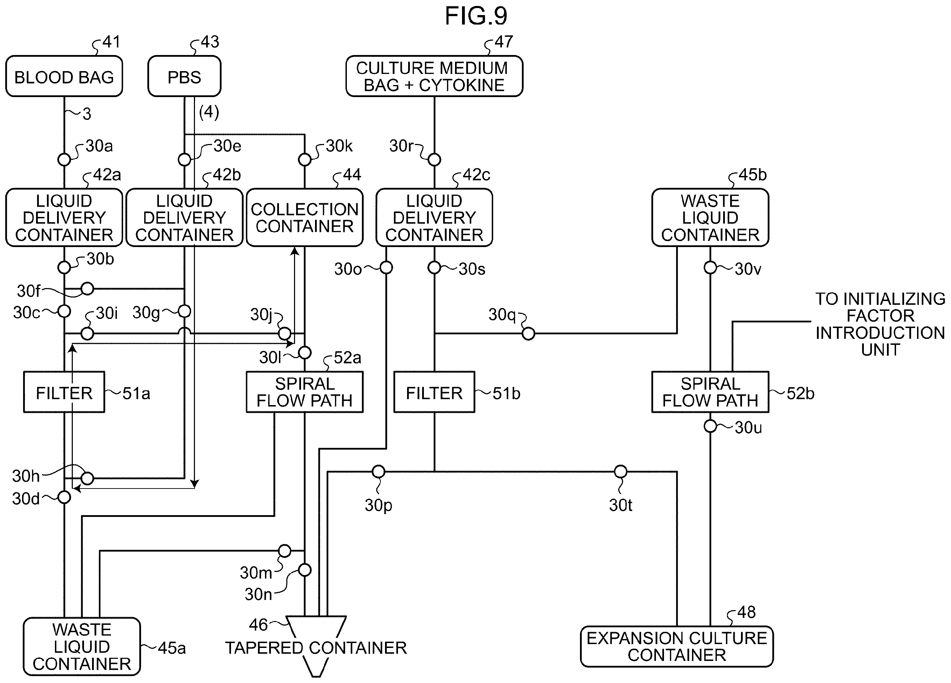

Subsequently, the cell collection step will be described. FIG. 9 is a view illustrating an example of a liquid delivery route in the cell collection step. In FIG. 9, the liquid delivery route in the cell collection step is represented by (4) and arrows.

At the cell collection step, the valve opening/closing apparatus 12 first sets the valves 30c, 30d, and 30f in the closed state. Thereafter, the liquid delivery apparatus 11 depressurizes the liquid delivery container 42b. This allows PBS to be delivered from the chemical solution bag 43 to the liquid delivery container 42b.

After the PBS is delivered into the liquid delivery container 42b, the valve opening/closing apparatus 12 sets the valves 30g, 30h, 30i, and 30j in the open state. Thereafter, the liquid delivery apparatus 11 pressurizes the liquid delivery container 42b. At the cell collection step, PBS is delivered from the liquid delivery container 42b through the filter 51a to the collection container 44. This allows the cells rinsed with PBS to be collected from the filter 51a and to be temporarily stored in the collection container 44.

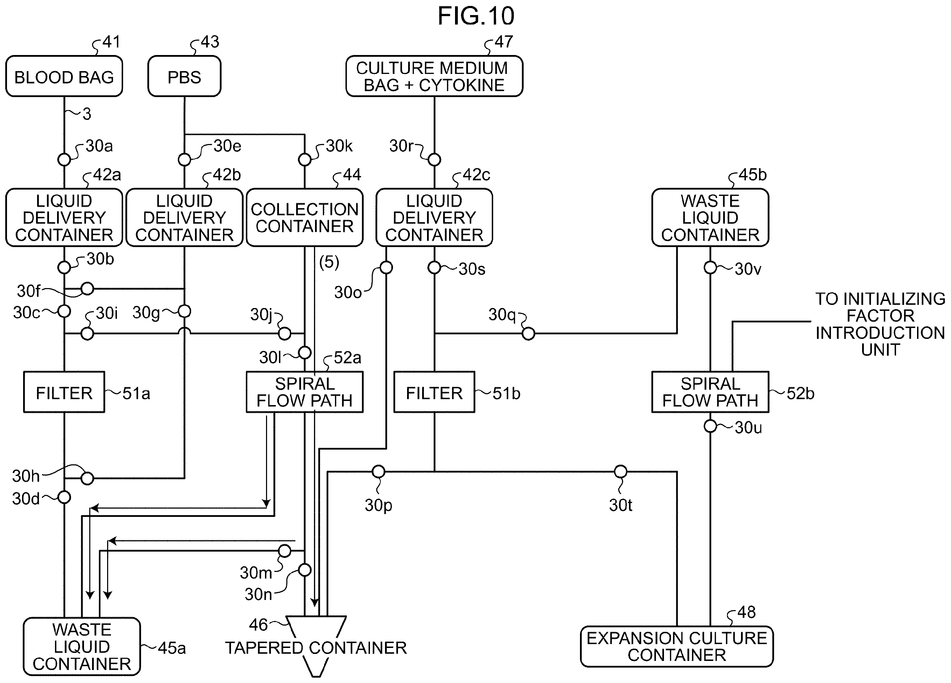

Subsequently, the first classification step will be described. FIG. 10 is a view illustrating an example of the liquid delivery route in the first classification step. In FIG. 10, the liquid delivery route in the first classification step is represented by (5) and arrows.

First, the valve opening/closing apparatus 12 sets the valve 30j in the closed state and the valves 30l and 30n in the open state. Thereafter, the liquid delivery apparatus 11 pressurizes the collection container 44. This allows the PBS containing cells collected from the filter 51a to be separately delivered from the collection container 44 through the spiral flow path 52a to the waste liquid container 45a and the tapered container 46.

The spiral flow path 52a and the spiral flow path 52b is an example of a device that selects objects in liquid by the action of flow. The spiral flow path 52a and the spiral flow path 52b is also called a helical flow path.

The spiral flow path 52a classifies the specific cells from the PBS containing cells collected from the filter 51a based on the size of the cells. The spiral flow path 52a is configured so as to classify particles having a size equal to or larger than the separation threshold A by controlling the flow rate. In this case, a plurality types of cells that are classified as particles having a size equal to or larger than the separation threshold A are an example of the first cell group.

Here, at the blood filtering step described above, particles having a size equal to or larger than the separation threshold A are collected by the filter 51a. However, the particle size through the selection by the filter is rougher than that of the first classification step using the spiral flow path. Therefore, the cells collected by filter 51a may contain particles having a size smaller than the separation threshold A. In the present embodiment, performing the treatment of the first classification step allows red blood cells and platelets, which are particles having a size smaller than the separation threshold A, to be more appropriately removed from the sample.

In the present embodiment, the red blood cells and the platelets are removed from the sample by the filter and the spiral flow path. However, the red blood cells and the platelets may be removed from the sample by only any one of the filter and the spiral flow path. Methods other than the methods described above may be used as long as the methods are capable of selecting the specific cells based on the cell size.

In the present embodiment, the cells having a size equal to or larger than the separation threshold A flow into the tapered container 46. The cells having a size smaller than the separation threshold A are discharged into the waste liquid container 45a.

The valve opening/closing apparatus 12 also allows the cells classified as particles having a size equal to or larger than the separation threshold A to be discharged into the waste liquid container 45a by setting the valve 30n in the closed state and the valve 30m in the open state.

For example, until a predetermined time elapses from the start of liquid delivery, the cells classified as particles having a size equal to or larger than the separation threshold A may be discharged into the waste liquid container 45a as described above. This allows the cells that are classified as particles having a size equal to or larger than the separation threshold A to flow into the tapered container 46 after waiting for the classification to stabilize.

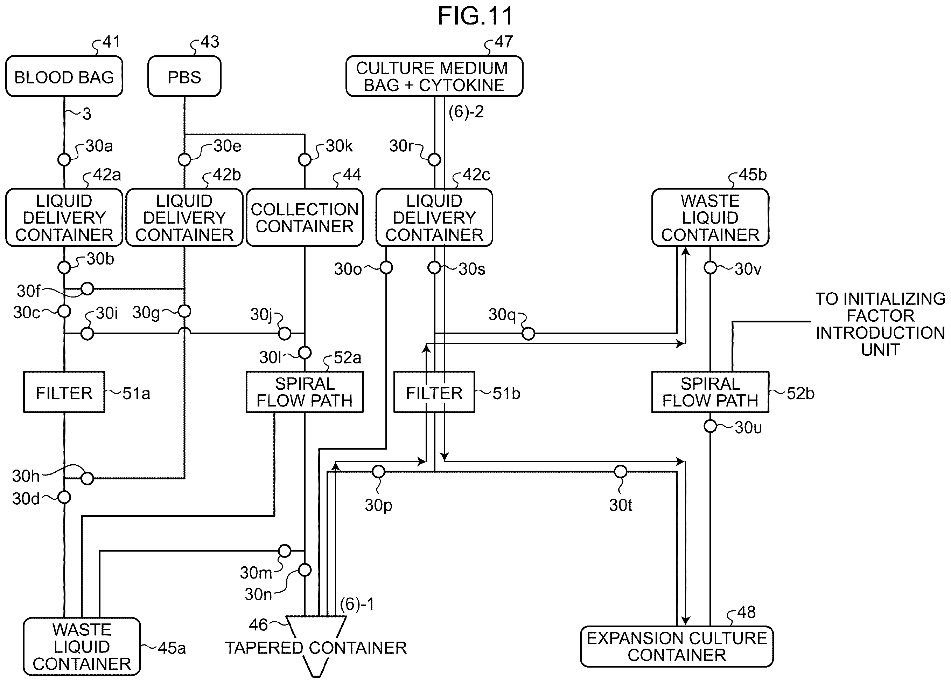

Subsequently, the expansion culture step will be described. FIG. 11 is a view illustrating an example of a liquid delivery route in the expansion culture step. In FIG. 11, the liquid delivery routes in the expansion culture step are indicated by (6)-1, (6)-2, and arrows.

First, the valve opening/closing apparatus 12 sets the valve 30n in the closed state and the valves 30o, 30p, and 30q in the open state. Thereafter, the liquid delivery apparatus 11 pressurizes the liquid delivery container 42c. This allows the tapered container 46 connected to the liquid delivery container 42c by the pipe 3 to be also pressurized. Pressurizing the tapered container 46 allows PBS containing the specific cells to be delivered from the tapered container 46 to the filter 51b. The PBS containing particles that passes through the filter 51b is delivered to the waste liquid container 45b ((6)-1).

Here, the filter 51b is configured so as to pass particles having a size smaller than the separation threshold A and so as to capture particles having a size equal to or larger than the separation threshold A. Therefore, the cell group containing the specific cells, which are particles having a size equal to or larger than the separation threshold A, is captured by the filter 51b, while PBS containing particles having a size smaller than the separation threshold A transfers to the waste liquid container 45b.

After the PBS containing particles having a size smaller than the separation threshold A is delivered from the tapered container 46 to the waste liquid container 45b, the valve opening/closing apparatus 12 sets the valves 30o, 30p, and 30q in the closed state and the valve 30r in the open state. Thereafter, the liquid delivery apparatus 11 depressurizes the liquid delivery container 42b.

This allows the culture medium and cytokines to be delivered from the culture medium bag 47 to the liquid delivery container 42c. As described above, depressurizing the inflow side of the liquid delivery container 42c by the liquid delivery apparatus 11 allows the liquid delivery control to be performed on the culture medium and cytokines stored in the culture medium bag 47.

After the culture medium and cytokines are delivered into the liquid delivery container 42c, the valve opening/closing apparatus 12 sets the valves 30s and 30t in the open state. Thereafter, the liquid delivery apparatus 11 pressurizes the liquid delivery container 42c. This allows the culture medium and cytokines to be delivered from the liquid delivery container 42c through the filter 51b to the expansion culture container 48 ((6)-2).

Therefore, the cell group containing the specific cells captured on the filter 51b is swept away by the culture medium and cytokines and transferred to the expansion culture container 48 with the culture medium and cytokines. In other words, the cell group including the specific cells is collected in the expansion culture container 48 in a state where the cell group is suspended in the culture medium and cytokines by the above liquid delivery operation.

In the present embodiment, the cell group containing the specific cells collected in the expansion culture container 48 is expansion-cultured for 5 days. This allows the cells (target cells) that are the target for the initializing factor introduction and are contained in the specific cells to increase in size and to proliferate. In the present embodiment, the CD34-positive cells increase in size and proliferate.

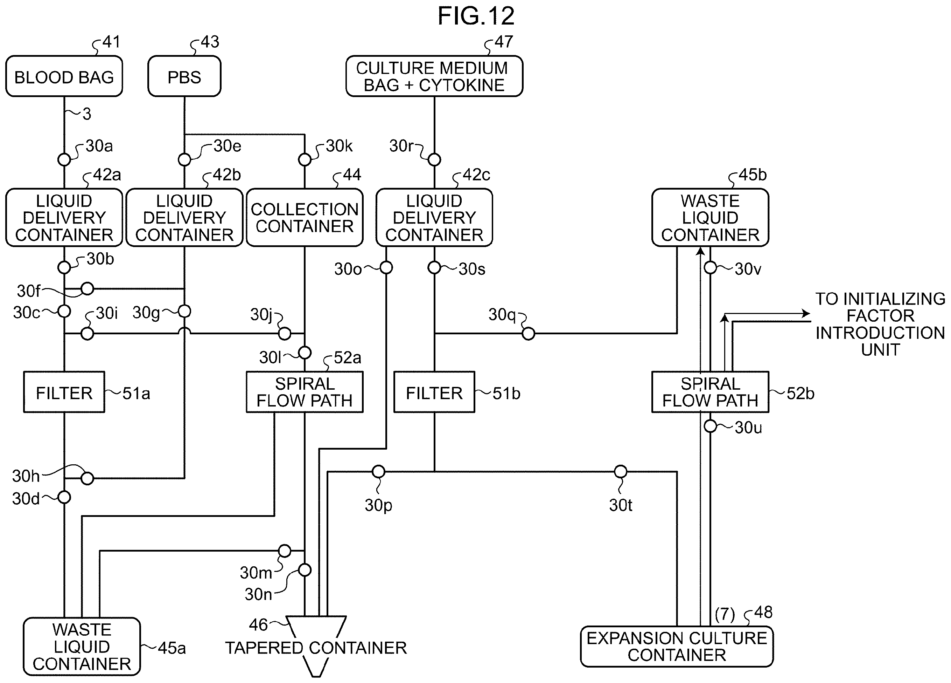

Subsequently, the second classification step will be described. FIG. 12 is a view illustrating an example of a liquid delivery route in the second classification step. In FIG. 12, the liquid delivery route in the second classification step is represented by (7) and arrows.

First, the valve opening/closing apparatus 12 sets the valves 30u and 30v in the open state. Thereafter, the liquid delivery apparatus 11 pressurizes the liquid delivery container 42c. This allows the expansion culture container connected to the liquid delivery container 42c by the pipe 3 to be also pressurized. Pressurizing the expansion culture container 48 allows the cell suspension liquid (culture medium) containing the expansion-cultured specific cells to be separately delivered from the expansion culture container 48 through the spiral flow path 52b to the waste liquid container 45b and the pipe 3 toward the initializing factor introduction unit (not illustrated) in which the treatment related to the initializing factor introduction process is performed.

The spiral flow path 52b classifies cells that have high possibility to be the target cells and other blood cells from the cell suspension liquid containing the expansion cultured specific cells based on the size of the cells. The spiral flow path 52b is a spiral flow path of the same design as the spiral flow path 52a. However, the flow rate of the liquid delivery during the classification differs between the first classification step and the second classification step. Here, the flow path 300 may be configured so that the second classification step can be performed using the spiral flow path 52a without providing the spiral flow path 52b.

The spiral flow path 52a is configured so as to classify particles having a size equal to or larger than the separation threshold B by controlling the flow rate. In this case, the separation threshold B is an example of the second threshold. A plurality of types of cells that are classified as particles having a size equal to or larger than the separation threshold B are an example of the second cell group.

Here, the separation threshold B is a value larger than the separation threshold A. In the case where the iPS cells are prepared from blood, the separation threshold B is preferably set at about 7 µm to 9 µm in order to properly remove lymphocytes present in the cell suspension liquid containing the expansion-cultured specific cells.

In the present embodiment, although lymphocytes are removed from the sample using the spiral flow path, the filter may be used to remove lymphocytes from the sample. The filter and the spiral flow path may also be used to remove lymphocytes.

In the present embodiment, the cells having a size equal to or larger than the separation threshold B are delivered into the pipe 3 toward the initializing factor introduction unit. The cells having a size smaller than the separation threshold B are discharged into the waste liquid container 45b.

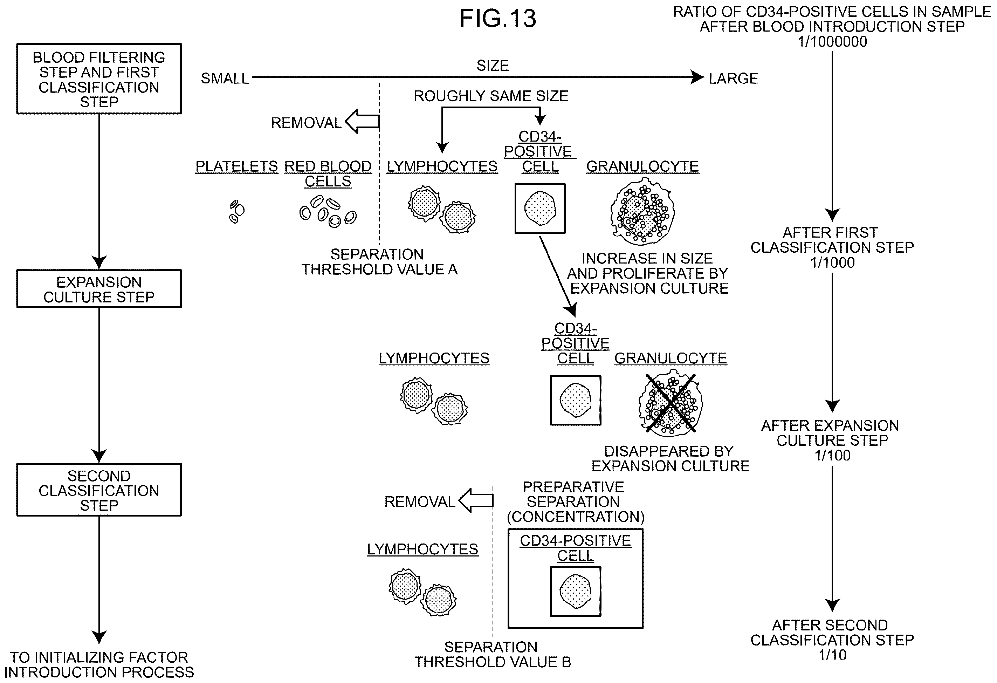

Performing the seven-step treatments described above allows the cell preparation apparatus 1 according to the present embodiment to efficiently concentrate the CD34-positive cells. Hereinafter, FIG. 13 is used to describe the reason why the cell preparation apparatus 1 according to the present embodiment can efficiently concentrate the CD34-positive cells. FIG. 13 is a view illustrating an example of the relationship between each step of the iPS cell preparation process and the ratio of the CD34-positive cells in the sample.

As illustrated in FIG. 13, the ratio of the CD34-positive cells in the sample after the blood introduction step is about 1/1,000,000. From here, the cell preparation apparatus 1 performs the blood filtering step and the first classification step, whereby red blood cells and platelets, which are cells having sizes smaller than the separation threshold A, are removed from the sample. This allows the ratio of the CD34-positive cells in the sample to be about 1/1,000.

Here, as illustrated in FIG. 13, in the cells selected at the blood filtering step and the first classification step, lymphocytes that are roughly the same size as the size of the CD34-positive cells are included. At this stage, lymphocytes that are roughly the same size as the size of the CD34-positive cells cannot be removed based on the cell size. Therefore, the cell preparation apparatus 1 according to the present embodiment performs the treatment of the expansion culture step.

At the expansion culture step, the cell preparation apparatus 1 cultures the cells selected at the blood filtering step and the first classification step under conditions suitable for the CD34-positive cells. As an example, the cell preparation apparatus 1 performs the expansion culture of a plurality of cells selected at the blood filtering step and the first classification step for 5 days under conditions of StemSpanSFEM (manufactured by STEMCELL Technologies) with adding cytokines.

This allows the CD34-positive cells in the cells selected at the blood filtering step and the first classification step to increase in size and proliferate. In addition, the expansion culture step is performed under conditions suitable for the CD34-positive cells, so that lymphocytes do not increase in size and proliferate. Therefore, lymphocytes that are roughly the same size as the size of the CD34-positive cells before the expansion culture become smaller than the CD34-positive cells after the expansion culture.

Furthermore, granulocytes contained in the cells selected at the blood filtering step and the first classification step disappear from the sample because the granulocytes can no longer retain the structure when the culture is performed under conditions suitable for the CD34-positive cells. In other words, performing the treatment at the expansion culture step allows granulocytes contained in the cells selected at the blood filtering step and the first classification step to be removed from the sample. This allows the ratio of the CD34-positive cells in the sample to be about 1/100.

From this stage, the cell preparation apparatus 1 removes lymphocytes, which are cells having a size smaller than the separation threshold B, from the sample by performing the second classification step. This allows the ratio of the CD34-positive cells in the sample to be about 1/10.

As described above, the cell preparation apparatus 1 according to the present embodiment performs the second classification step, in which the separation threshold is changed from the first classification step, after the cells selected at the blood filtering step and the first classification step are expansion-cultured, whereby lymphocytes that are roughly the same size as the size of the CD34 positive cells before the expansion culture can be removed from the sample.

As described above, the cell preparation apparatus 1 according to the present embodiment described above selects the cell group composed of a plurality of types of cells having a size equal to or larger than the separation threshold A from a plurality of cells contained in the liquid sample, performs expansion cultures of the target cells contained in the selected cell group and serving as the object for concentration, and selects the cell group having a size equal to or larger than the separation threshold B, which is larger than the separation threshold A from the expansion-cultured cell group.

In the cell preparation apparatus 1 according to the present embodiment, this allows only the target cells to increase in size and to proliferate among a plurality of types of cells selected as cells having a size equal to or larger than the separation threshold A by performing the expansion culture in an environment suitable for the target cells, for example, in the case where the cells to be selected (target cells) are rare cells. Therefore, the cell preparation apparatus 1 according to the present embodiment can efficiently remove unnecessary cells that are roughly the same size as the size of the target cells in a state before the expansion culture from the sample by selecting the cell group having a size equal to or larger than the separation threshold B after the expansion culture. In other words, according to the cell preparation apparatus 1 according to the present embodiment, rare cells in biological samples can be efficiently concentrated.

Incidentally, in the present embodiment, the CD34-positive cells are included among the types of cells selected as the cell group of which cell size is equal to or larger than the separation threshold B as described above. The CD34-positive cells are rare cells that are the target for initiation factor introduction in the case where the iPS cells are prepared from blood. Therefore, the collection of the CD34-positive cells using antigen-antibody reactions may have conventionally been used for the purpose of performing efficient initializing factor induction. For example, an anti-CD34 antibody attached to porcelain beads is added to blood to react with the CD34-positive cells, and thereafter the porcelain beads are attracted by a magnet to collect the CD34-positive cells bound to the porcelain beads through the anti-CD34 antibody. In addition, for example, an anti-CD34 antibody attached to a fluorescent dye is added to blood to react with the CD34-positive cells, and thereafter flow cytometry is used to select and collect the CD34-positive cells bound to the fluorescent dye through the anti-CD34 antibody. However, in the methods described above, impurities such as unreacted porcelain beads and fluorescent dyes may remain in the sample. In general, a mechanism to perform the process of collecting the CD34-positive cells using the above methods is difficult to be installed to a cell preparation apparatus that automatically performs a series of processes such as blood cell introduction, blood cell separation, expansion culture, initializing factor introduction, culture, passaging, and stocking. In contrast, in the cell preparation apparatus 1 according to the present embodiment, only CD34-positive cells can increase in size and proliferate among the types of cells of which cell sizes are equal to or larger than the separation threshold A by performing the expansion culture in an environment suitable for the CD34-positive cells after selecting a cell group of which cell size is equal to or larger than the separation threshold A. Selecting the cell group of which cell size is equal to or larger than the separation threshold B after the expansion culture allows lymphocytes that are roughly the same size as the size of the CD34-positive cells in a state before the expansion culture can be removed from the sample. In other words, according to the cell preparation apparatus 1 according to the present embodiment, the sample containing many CD34-positive cells can be efficiently obtained without adding reagents not directly related to the preparation of the iPS cells to the sample or complicating the apparatus configuration.

The embodiments described above can also be implemented with appropriate modifications by modifying some of the configurations or functions of the cell preparation apparatus 1. Therefore, hereinafter, modified examples according to the embodiments described above will be described as other embodiments. Hereinafter, the points that differ from the embodiments described above will be mainly described, and a detailed description of the points in common with those already explained will be omitted. The modified examples described below may be implemented individually or implemented in combination as appropriate.

### Modified Example 1

The embodiments described above describe aspects of concentrating the CD34-positive cells in blood. However, the target cells for the object for concentration may be cells other than the CD34-positive cells. The target cells may be any types of cells as long as the types of cells achieve size differences between the target cells and non-target cells by performing the expansion culture.

According to the present modified example, rare cells other than the CD34-positive cells can be efficiently concentrated.

### Modified Example 2

The embodiments described above describe an aspect in which a series of treatments related to the blood introduction process, the blood cell separation process, the expansion culture process, the initializing factor introduction process, the culture process, the passaging process, and the stocking process according to the preparation of the iPS cells are performed in a single cell preparation apparatus 1. However, a plurality of apparatuses may be used for the treatments related to these processes. In this case, each process may be handled by different apparatuses, or there may be an apparatus handling a plurality of processes.

The embodiments described above describe an aspect in which all of the treatments related to the processes described above are automatically performed by the cell preparation apparatus 1. However, some or all treatments related to the processes described above may be manually performed.

According to the present modified example, rare cells in biological samples can be efficiently concentrated in a manner suitable for the equipment and environment of a facility in which the cells are prepared using the biological samples as the material.

As described above, according to at least one embodiment or modified example described above, the rare cells in biological samples can be efficiently concentrated.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

### Notes

With respect to the embodiments described above, the following notes are further disclosed.

### Note 1

A cell concentration apparatus according to an embodiment includes: a first selection unit configured to collect cells having a size equal to or larger than a first threshold from a plurality of cells contained in a liquid sample as a first cell group; a culture unit configured to perform expansion culture of target cells included in the collected first cell group and serving as an object for concentration; and a second selection unit configured to select, from the expansion-cultured first cell group, cells having a size equal to or larger than a second threshold larger than the first threshold and collect the cells as a second cell group.

### Note 2

The liquid sample may be blood.

### Note 3

The target cells may be CD34-positive cells, the first selection unit may be configured to select a plurality of types of cells including the CD34-positive cells as the first cell group, the culture unit may be configured to perform expansion culture of the CD34-positive cells, and the second selection unit may be configured to select a plurality of cells including the expansion-cultured CD34-positive cells as the second cell group.

### Note 4

The culture unit may be configured to increase a size of the target cells and to proliferate the target cells by performing expansion culture in an environment corresponding to the target cells.

### Note 5

The first selection unit may be at least one of a filter passing particles having a particle size smaller than the first threshold and capturing particles having a particle size equal to or larger than the first threshold and a spiral flow path classifying particles based on a particle size, and the second selection unit may be at least one of a filter passing particles having a particle size smaller than the second threshold and capturing particles having a particle size equal to or larger than the second threshold and the spiral flow path.

### Note 6

The first selection unit may include the spiral flow path and is configured to select the first cell group by classifying particles having a particle size equal to or larger than the first threshold by fluid having a predetermined flow rate flowed into the spiral flow path, and the second selection unit may include the spiral flow path and is configured to select the second cell group by classifying particles having a particle size equal to or larger than the second threshold by fluid having a flow rate different from the predetermined flow rate using the spiral flow path.

### Note 7

A method for concentrating cells according to an embodiment includes: a first selection step of collecting cells having a size equal to or larger than a first threshold from a plurality of cells contained in a liquid sample as a first cell group; a culture step of performing expansion culture of target cells included in the collected first cell group and serving as an object for concentration, and a second selection step of selecting, from the expansion-cultured first cell group, cells having a size equal to or larger than a second threshold larger than the first threshold and collecting the cells as a second cell group.

### Note 8

The liquid sample may be blood.

### Note 9

The target cells may be CD34-positive cells; the first selection step may select a plurality of types of cells including the CD34-positive cells as the first cell group; the culture step may perform expansion culture of the CD34-positive cells, and the second selection step may select a plurality of cells including the expansion-cultured CD34-positive cells as the second cell group.

### Note 10

The culture step may increase a size of the target cells and proliferates the target cells by performing expansion culture in an environment corresponding to the target cells.

### Note 11

The first selection step may select the first cell group using at least one of a filter passing particles having a particle size smaller than the first threshold and capturing particles having a particle size equal to or larger than the first threshold and a spiral flow path classifying particles based on a particle size, and the second selection step may select the second cell group using at least one of a filter passing particles having a particle size smaller than the second threshold and capturing particles having a particle size equal to or larger than the second threshold and the spiral flow path.

### Note 12

The first selection step may select the first cell group by classifying particles having a particle size equal to or larger than the first threshold by controlling a flow rate of fluid flowed into the spiral flow path, and the second selection step may select the second cell group by classifying particles having a particle size equal to or larger than the second threshold by changing the flow rate from the flow rate of the first selection step using the spiral flow path used at the first selection step.

## Claims

1. A cell concentration apparatus (1) comprising:
a first selection unit (51a,51b) configured to collect cells having a size equal to or larger than a first threshold from a plurality of cells contained in a liquid sample as a first cell group;
a culture unit (48) configured to perform expansion culture of target cells included in the collected first cell group and serving as an object for concentration; and
a second selection unit (52a,52b) configured to select, from the expansion-cultured first cell group, cells having a size equal to or larger than a second threshold larger than the first threshold and collect the cells as a second cell group.

2. The cell concentration apparatus (1) according to claim 1, wherein the liquid sample is blood.

3. The cell concentration apparatus (1) according to claim 1 or 2, wherein
the target cells are CD34-positive cells,
the first selection unit (51a,51b) is configured to select a plurality of types of cells including the CD34-positive cells as the first cell group;
the culture unit (48) is configured to perform expansion culture of the CD34-positive cells, and
the second selection unit (52a,52b) is configured to select a plurality of cells including the expansion-cultured CD34-positive cells as the second cell group.

4. The cell concentration apparatus (1) according to any one of claims 1 to 3, wherein the culture unit (48) is configured to increase a size of the target cells and to proliferate the target cells by performing expansion culture in an environment corresponding to the target cells.

5. The cell concentration apparatus (1) according to any one of claims 1 to 4, wherein
the first selection unit (51a,51b) is at least one of a filter passing particles having a particle size smaller than the first threshold and capturing particles having a particle size equal to or larger than the first threshold and a spiral flow path classifying particles based on a particle size, and
the second selection unit (52a,52b) is at least one of a filter passing particles having a particle size smaller than the second threshold and capturing particles having a particle size equal to or larger than the second threshold and the spiral flow path.

6. The cell concentration apparatus (1) according to claim 5, wherein
the first selection unit (51a,51b) comprises the spiral flow path and is configured to select the first cell group by classifying particles having a particle size equal to or larger than the first threshold by fluid having a predetermined flow rate flowed into the spiral flow path, and
the second selection unit (52a,52b) comprises the spiral flow path and is configured to select the second cell group by classifying particles having a particle size equal to or larger than the second threshold by fluid having a flow rate different from the predetermined flow rate using the spiral flow path.

7. A method for concentrating cells, the method comprising:
a first selection step of collecting cells having a size equal to or larger than a first threshold from a plurality of cells contained in a liquid sample as a first cell group;
a culture step of performing expansion culture of target cells included in the collected first cell group and serving as an object for concentration, and
a second selection step of selecting, from the expansion-cultured first cell group, cells having a size equal to or larger than a second threshold larger than the first threshold and collecting the cells as a second cell group.

8. The method for concentrating cells according to claim 7, wherein the liquid sample is blood.

9. The method for concentrating cells according to claim 7 or 8, wherein
the target cells are CD34-positive cells;
the first selection step selects a plurality of types of cells including the CD34-positive cells as the first cell group;
the culture step performs expansion culture of the CD34-positive cells, and
the second selection step selects a plurality of cells including the expansion-cultured CD34-positive cells as the second cell group.

10. The method for concentrating cells according to any one of claims 7 to 9, wherein the culture step increases a size of the target cells and proliferates the target cells by performing expansion culture in an environment corresponding to the target cells.

11. The method for concentrating cells according to any one of claims 7 to 10, wherein
the first selection step selects the first cell group using at least one of a filter passing particles having a particle size smaller than the first threshold and capturing particles having a particle size equal to or larger than the first threshold and a spiral flow path classifying particles based on a particle size, and
the second selection step selects the second cell group using at least one of a filter passing particles having a particle size smaller than the second threshold and capturing particles having a particle size equal to or larger than the second threshold and the spiral flow path.

12. The method for concentrating cells according to claim 11, wherein
the first selection step selects the first cell group by classifying particles having a particle size equal to or larger than the first threshold by controlling a flow rate of fluid flowed into the spiral flow path, and
the second selection step selects the second cell group by classifying particles having a particle size equal to or larger than the second threshold by changing the flow rate from the flow rate of the first selection step using the spiral flow path used at the first selection step.
